# EUROPÄISCHE PATENTANMELDUNG

(11) **EP 4 458 399 A1**
(43) Veröffentlichungstag der Anmeldung: **06.11.2024**
(21) Anmeldenummer: 23171665.5
(22) Anmeldetag: 04.05.2023
(51) Int. Cl.: A61M 25/00, A61B 17/00

(54) **VORRICHTUNG ZUR APPLIKATION EINES ALS FLUID AUSGEBILDETEN THERAPEUTISCHEN MITTELS**

(71) Anmelder: FUJIFILM Corporation, Tokyo 107-0052 (JP)
(72) Erfinder: Schrader, Matthias, 90491 Nürnberg (DE); Dieter, Johannes, 96050 Bamberg (DE)
(74) Vertreter: Gosdin, Carstensen & Partner Patentanwälte Partnerschaftgesellschaft mbB

(57) **Zusammenfassung**

Eine Vorrichtung (1) soll zur Applikation eines therapeutischen, als Fluid ausgebildeten Mittels an einer Gewebewand dienen, die eine Körperhöhle umschließt. Dabei ist die Vorrichtung (1) an einem in die Körperhöhle einführbaren Katheter angeordnet und nach dem Einführen in Bezug auf den Katheter zum Berühren der Gewebewand radial aufspreizbar, wobei das über den Katheter zugeführte Fluid über Austrittsöffnungen (10) an die Gewebewand gelangt. Zur Schaffung einer Vorrichtung, mit der im Rahmen einer endoskopischen Behandlung Mittel appliziert werden können, soll der Katheter als Tubus (5) einer endoskopischen Einrichtung (6) ausgebildet sein, wobei der Tubus (5) an seinem distalen Ende die Vorrichtung (1) aufnimmt und ein Lumen (4) aufweist, in welchem sich ein mechanisches Steuerelementelement (2) erstreckt. Durch Längsbewegungen des Steuerelementelement (2), die proximal über ein Betätigungselement erzeugt werden, wird die Vorrichtung (1) zwischen einem Zustand kleinster radialer Erstreckung und einem Zustand größter radialer Erstreckung verstellt, wobei sie zumindest in ihrem Abschnitt, der die Gewebewand im aufgespreizten Zustand berührt, die Austrittsöffnungen (10) für das Fluid aufweist.

## Beschreibung

Die Erfindung betrifft eine Vorrichtung zur Applikation eines als Fluid ausgebildeten therapeutischen Mittels an einer Gewebewand, die eine Körperhöhle umschließt, wobei die Vorrichtung an einem in die Körperhöhle einführbaren Katheter angeordnet und nach dem Einführen in Bezug auf den Katheter zum Berühren der Gewebewand radial aufspreizbar ist und wobei das über den Katheter zugeführte Fluid über Fluidaustrittsöffnungen an die Gewebewand gelangt.

Zur wirkungsvollen Behandlung bestimmter Krankheiten, die in Körperhöhlen und Organe auftreten, kann es zweckmäßig sein, therapeutische Mittel auf das von der Krankheit betroffene Gewebe, also eine die Körperhöhle umgebende Gewebewand oder ein an diese angrenzendes Organ aufzubringen. Auf diese Weise kann das Mittel, das häufig wegen Nebenwirkungen für eine orale Verabreichung nicht geeignet ist, unmittelbar und gezielt in das erkrankte Gewebe eingebracht werden, um dieses zu behandeln.

Ein weiteres medizinisches Problem, bei dem eine Gewebewand einer Körperhöhle zu behandeln ist, sind gastrointestinale Blutungen, die mit hoher Inzidenz als akute Ulkusblutungen im oberen Gastrointestinaltrakt auftreten. Diese erfordern eine sofortige und zügige Behandlung. Im Rahmen einer Therapie zur Hämostase, bei der möglichst auf einen operativen Eingriff verzichtet werden soll. Hierfür werden häufig endoskopische Vorrichtungen verwendet, mit denen medizinische Clips in der Gewebewand zum Wundverschluss und somit Stillung der Blutung platziert werden. Starke Blutungen können im Gastrointestinaltrakt insbesondere nach einer endoskopischen Resektion von Gewebe, wie beispielsweise nach einer Polypektomie auftreten. Weiterhin kann es sich bei Blutungen im menschlichen Körper auch um solche im unteren Gatrointestinaltrakt handeln, die ebenfalls mittels endoskopisch applizierter medizinischer Clips gestillt werden können, wobei das in Rahmen einer Koloskopie erfolgt. Insgesamt ergibt sich beim Einsatz einer endoskopischen Therapie eine geringe Rezidivblutungsrate, sofern es sich nicht um perakute Blutungen mit massiver Aktivität handelt.

Außerdem können zur endoskopischen Hämostase neben der Verwendung von Clips auch eine Injektionstherapie oder eine Thermotherapie eingesetzt werden. Bei der Thermotherapie werden zur Stillung der Blutung thermische Mittel verwendet, wie z. B. das Kauterisieren einer oder mehrerer Stellen im Gewebe. Dabei kann das umgebende parenchymale Gewebe geschädigt werden.

Eine Vorrichtung zur Applikation eines therapeutischen Mittels, das als Fluid ausgebildet ist, an einer eine Körperöffnung umschließenden Gewebewand der im Oberbegriff des Patentanspruchs 1 genannten Gattung ist aus der US 2019/0232036 A1 bekannt. Danach wird eine minimalinvasive Vorrichtung verwendet, um eine Hämostase des Gewebes einer Prostatakapsel nach einer in dieser ebenfalls minimalinvasiv durchgeführten Geweberesektion zu erzielen. Die Vorrichtung besteht im Wesentlichen aus einem Katheter, einem distalen Ballon und einem proximalen Ballon und kann durch die Harnröhre des Patienten eingeführt werden, so dass sich der distale Ballon in der Blase, dem Blasenhals unmittelbar benachbart, und der proximale Ballon innerhalb der Prostatakapsel befinden. Proximal soll das Innere der Prostatakapsel gegenüber dem Harnleiter durch den in diesem verlaufenden Katheter abgedichtet sein.

Wenn nun der distale Ballon über ein entsprechendes Lumen, das innerhalb des Katheters verläuft, mit einem Druckmittel befüllt und somit aufgeblasen wird, ist das Innere der Prostatakapsel auch distal gegenüber der Blase abgedichtet, wobei sich der distale Ballon dichtend an den Blasenhals anlegt. Anschließend wird dann das den Wundverschluss bewirkende flüssige Hämostasemittel über ein weiteres Lumen in die Resektionshöhle der Prostatakapsel geleitet, wobei es über eine Infusionsöffnung, die in der Nähe des distalen Endes des sich im Inneren der Prostatakapsel befindlichen Katheters angeordnet ist, in die Resektionshöhle eingebracht wird. In einem weiteren Schritt soll der proximale Ballon über ein weiteres Lumen aufgeblasen werden, um das Hämostasemittel auf die Gewebewand aufzutragen und über die vom proximalen Ballon übertragene Druckkraft gegen die Gewebewand zu pressen.

Bei einer weiteren aus diesem Dokument bekannten Ausführungsform soll die Zufuhr des flüssigen Hämostasemittels über den proximalen Ballon erfolgen, wobei dieser mit gleichmäßig über seinen Umfang verteilten Poren versehen ist, über die das in den Ballon strömende Hämostasemittel im Inneren der Prostatakapsel verteilt wird. Es ist vorgesehen, dass das unter Druck in den Ballon einströmende Hämostasemittel auch dafür sorgt, dass der Ballon expandiert und sich an die Gewebewand legt, um zu bewirken, dass das Hämostasemittel gleichmäßig an der Gewebewand abgegeben und gegen diese gepresst wird.

Es ist Aufgabe der vorliegenden Erfindung, eine Vorrichtung zur Applikation eines therapeutischen Mittels, das als Fluid ausgebildet ist, an einer eine Körperöffnung umschließenden Gewebewand der im Oberbegriff des Patentanspruchs 1 angegebenen Gattung derart weiter zu entwickeln, dass mit dieser eine endoskopische Behandlung durchgeführt werden kann.

Diese Aufgabe wird, ausgehend vom Oberbegriff des Patentanspruchs mit dessen kennzeichnenden Merkmalen gelöst. Die vom Patentanspruch 1 abhängigen Patentansprüche beinhalten erfindungsgemäße Weiterbildungen dieser Lösung.

Danach ist eine Vorrichtung vorgesehen, mittels der ein therapeutisches als Fluid ausgebildetes Mittel an einer Gewebewand, die eine Körperhöhle umschließt, applizierbar ist. Die Vorrichtung ist an einem in die Körperhöhle einführbaren Katheter angeordnet und nach dem Einführen in Bezug auf den Katheter zum Berühren der Gewebewand radial aufspreizbar. Das über den Katheter zugeführte Fluid gelangt über Fluidaustrittsöffnungen an die Gewebewand.

Erfindungsgemäß soll der Katheter als Tubus einer endoskopischen Einrichtung ausgebildet sein, wobei der Tubus an seinem distalen Ende die Vorrichtung aufnimmt. Vorzugsweise ist diese endoskopische Einrichtung für Behandlungen im oberen oder unteren Gastrointestinaltrakt vorgesehen. Der Tubus weist dabei ein Lumen auf, in welchem sich ein mechanisches Steuerelement erstreckt. Mittels eines Betätigungselements, das proximal am Steuerelement angreift und manuell betätigbar ist, werden Längsbewegungen des Steuerelements bewirkt, durch die die Vorrichtung zwischen einem Zustand kleinster radialer Erstreckung und einem Zustand größter radialer Erstreckung verstellbar ist.

Die Vorrichtung weist die Austrittsöffnungen für das Fluid zumindest in ihrem Abschnitt, der die Gewebewand im aufgespreizten Zustand berührt, auf. Durch eine Längsbewegung der Vorrichtung in proximaler Richtung, die vorzugsweise mittels einer entsprechenden Bewegung des Tubus` bewirkt wird, kann das aus den Austrittsöffnungen austretende therapeutische Mittel an einem vorgesehenen Bereich der Gewebewand aufgetragen werden, bei der es sich um die Magen- oder Darmmukosa handeln kann. In diese kann das Mittel einwirken.

Demgegenüber ist nach der gattungsbildenden US 2019/0232036 A1 eine minimalinvasive Behandlung der Prostata vorgesehen, wobei der an einem kurzen Katheter geführte proximale Ballon zum Auftragen des therapeutischen Mittels und zur Beaufschlagung des Bereichs einer Resektionswunde mit einer Kraft mittels eines gasförmigen oder flüssigen Druckmittels aufgeblasen wird. Das Mittel kann gemäß einer Ausführungsform zwar auch über Poren des proximalen Ballons zugeführt werden, wobei sich bei aufgeblasenem Ballon der Öffnungsquerschnitt der Poren vergrößert; damit können aber die zugeführte Menge und die Verteilung des therapeutischen Mittels nicht in ausreichendem Maße gesteuert werden.

In weiterer Ausgestaltung der Erfindung soll das Fluid ein Hämostasemittel zur Erzielung einer Hämostase an der Gewebewand nach einer an dieser vorgenommenen Geweberesektion sein. Eine derartige mittels eines Schneidwerkzeugs endoskopisch durchgeführte Geweberesektion kann beispielsweise im Gastrointestinaltrakt vorgenommen werden. Mit der endoskopischen Einrichtung, die die Vorrichtung zur Applizierung des Hämostasemittels aufweist, können von dem die Behandlung durchführenden Arzt mit großer Distanz entfernt liegende operierte Bereiche erreicht und dort das Hämostasemittel auf das verletzte Gewebe aufgebracht werden, um die auftretenden Blutungen zu hemmen. Bei dem Hämostasemittel kann es sich beispielsweise um einen Fibrinkleber handeln. Das einen Arbeitskanal aufweisende Endoskop kann nach der Geweberesektion im Körper des Patienten verbleiben, und es wird die erfindungsgemäße Vorrichtung gemeinsam mit dem Tubus sowie dem vorzugsweise als Seil oder Draht ausgeführten Steuerelement in den Arbeitskanal eingeführt.

Weiterhin soll die in das Lumen eingezogene Vorrichtung in diesem Zustand radial federnd vorgespannt sein und nach ihrem Austritt aus dem Lumen, für den sie mittels des Steuerelements in distaler Richtung verschiebbar ist, in ihre radial aufgespreizte Stellung gelangen. Mit in das Lumen eingezogener erfindungsgemäßer Vorrichtung wird der Tubus im Arbeitskanal vorgeschoben, bis dessen distales Ende, das aus dem Arbeitskanal vorsteht, an der zu behandelnden Stelle der Körperöffnung positioniert ist. Wenn dann die Vorrichtung in distaler Richtung betätigt wird und aus dem distalen Ende des Tubus austritt, federt diese radial auf und legt sich mit ihren Endabschnitten an die Gewebewand an, um auf diese das flüssige therapeutische Mittel aufzutragen.

Nach einem Ausführungsbeispiel der Erfindung wird die Vorrichtung durch Mikrodrähte gebildet, die in ihrem aus dem Lumen ausgeschobenen Zustand, insgesamt eine kelchartige Kontur bildend, zueinander verlaufen. Die Mikrodrähte können so geformt und vorgespannt sein, dass sie sich im entspannten Zustand in einen Verlauf mit einem au-ßen liegenden Radius bewegen, in dem die distalen Enden am Gewebe anliegen. Alternativ dazu kann es sich auch um einen Verlauf mit innenliegendem Radius handeln, bei denen ein Abschnitt ihrer distalen Enden in Anlage am Gewebe gelangt. Dabei können innerhalb der Mikrodrähte Zuführkanäle verlaufen, deren Austrittsöffnungen zum Auftragen des Fluids auf die Gewebewand dienen. Die Austrittsöffnungen sind derart in den Mikrodrähten positioniert, dass das Mittel unmittelbar auf die zu behandelnde Stelle aufgetragen wird. Alternativ dazu können an den Mikrodrähten Zuführschläuche fixiert sein, die jeweils mit zumindest einer Austrittsöffnung zum Auftragen des Fluids auf die Gewebewand versehen sind.

Außerdem ist vorgesehen, dass die Zuführkanäle der Mikrodrähte oder die Zuführschläuche mit dem das Steuerelement umgebenden Tubus oder einem innerhalb des Lumens verschiebbar geführten, das Steuerelement umschließenden Hauptschlauch verbunden sind. Im Tubus ist dabei zumindest ein weiteres Lumen vorgesehen, über die die Vorrichtung mit dem therapeutischen Mittel versorgt wird. Alternativ dazu kann sich ein entsprechendes Lumen im Hauptschlauch befinden, von dem die Mikrodrähte und/oder Zuführschläuche ausgehen.

Bei einer weiteren Ausgestaltung der erfindungsgemäßen Vorrichtung können die Mikrodrähte über eine doppelwandige Folienanordnung miteinander verbunden sein, wobei das Fluid zwischen den beiden Folien geführt wird und über in der äußeren Folie vorgesehene Austrittsöffnungen auf die Gewebewand übertragbar ist. In diesem Fall wird das Mittel zwischen die beiden Folienlagen geleitet und tritt über die Austrittsöffnungen der äußeren Folienlage aus dem Zwischenraum aus. Damit lässt sich das Mittel in günstiger Weise auf die Gewebewand auftragen, und die gespannte doppellagige Folie kann Druck auf die Gewebewand ausüben. Alternativ dazu kann die die Form eines umgekehrten Trichters aufweisende Folie einlagig ausgebildet sein und an ihrem Rand in eine mit dem Katheter verbundene Wulst übergehen. In der Wulst und in dem trichterartigen Teil der Folie befinden sich in diesem Fall die Austrittsöffnungen für das Hämostasemittel, das über das Lumen des Katheters zugeführt und von der einen Boden bildenden trichterförmigen Folie zu den Austrittsöffnungen der Wulst geleitet wird, aber auch an dieser austreten kann. Durch das mittig an dem Boden angreifende mechanische Steuerelement lässt sich die Form der Wulst und somit der Druck auf die Gewebewand verändern.

Weiterhin soll nach einer erfindungsgemäßen Ausgestaltung der Vorrichtung das Hämostasemittel der Vorrichtung über das Lumen oder eine im Tubus verlaufende Leitung zuführbar sein, wobei die Vorrichtung durch eine hülsenartige Folie gebildet wird. Diese ist mit den Austrittsöffnungen für das Fluid versehen und verbindet Teilabschnitte des Katheters, die mittels des Steuerelements in axialer Richtung relativ zueinander bewegbar sind, miteinander. Das Steuerelement bewirkt eine Verformung der zwischen den Tubusabschnitten angeordneten hülsenartigen Folie, die sich wulstartig radial nach au-ßen ausformt und Druck auf die gewebewand ausübt, wodurch sich die Stillung einer Blutung herbeiführen lässt.

Außerdem ist vorgesehen, dass das Steuerelement als Draht oder Seil ausgebildet ist. Mit diesem im Lumen geführten Draht oder Seil, das proximal mit einem Betätigungselement verbunden ist, lässt sich die vorgesehene erfindungsgemäße Vorrichtung, die einen großen Abstand zu dem vom behandelnden Arzt zu bedienenden Betätigungselement einnimmt, mit technisch einfachen Mitteln aktuieren.

Schließlich kann die gemeinsam mit dem Tubus ausgebildete Vorrichtung zur Applikation des als Fluid ausgebildeten Mittels für eine Anordnung in einem Arbeitskanal eines medizinischen Endoskops vorgesehen sein. Die Vorrichtung lässt sich nach einer endoskopischen Untersuchung des Gastrointestinaltrakts und einer eventuell anschließend durchgeführten Resektion von Polypen mittels des bereits im Magen-Darmtrakt angeordneten Endoskops bis in den Bereich der Körperhöhle führen, in dem ein medizinisches Mittel verabreicht oder eine Hämostase durchgeführt werden soll.

Die Erfindung ist nicht auf die angegebene Kombination der Merkmale des unabhängigen Patentanspruchs 1 und der von diesem abhängigen Patentansprüche beschränkt. Es ergeben sich darüber hinaus weitere Möglichkeiten, einzelne Merkmale, insbesondere dann, wenn sie sich aus den Patentansprüchen, den zu diesen angegebenen Vorteilen, der nachfolgenden Beschreibung der Ausführungsbeispiele oder unmittelbar aus der Zeichnung ergeben, miteinander zu kombinieren. Außerdem soll die Bezugnahme der Patentansprüche auf die Zeichnung durch die Verwendung von Bezugszeichen den Schutzumfang der Patentansprüche auf keinen Fall auf die dargestellten Ausgestaltungsbeispiele beschränken.

Weitere Merkmale der Erfindung ergeben sich im Übrigen aus der nachfolgenden Beschreibung und aus der Zeichnung, in der vier Ausführungsbeispiele der Erfindung vereinfacht dargestellt sind.

Es zeigen:
- Figur 1: als schematische Darstellung einen Längsschnitt durch eine erfindungsgemäße Vorrichtung, die am distalen Ende eines Tubus' angeordnet ist, gemäß einem ersten Ausgestaltungsbeispiel, wobei die Vorrichtung durch kelchartig zueinander verlaufende Mikrodrähte und Zuführschläuche gebildet wird,
- Figur 2: ein weiteres Ausgestaltungsbeispiel als schematische Darstellung im Längsschnitt, bei dem die Mikrodrähte über eine doppelwandige Folienanordnung miteinander verbunden sind,
- Figur 3: eine alternative Ausgestaltung, bei der eine Folie einen Boden mit einer umlaufenden Wulst bildet und in der Wulst Austrittsöffnungen vorgesehen sind,
- Figur 4: ein weiteres Ausgestaltungsbeispiel als schematische Darstellung im Längsschnitt, bei dem die Vorrichtung durch eine Teilabschnitte des Tubus` verbindende hülsenartige Folie gebildet wird, wobei die Teilabschnitte mittels des Steuerelements in axialer Richtung aufeinander zu bewegt werden, so dass die Folie radial wulstartig nach außen geformt wird,
- Figur 5: einen Längsschnitt durch eine Körperhöhle eines menschlichen Körpers, bei der aufgrund einer Resektion eine Mukosa und weitere darunter liegende Gewebeschichten verletzt sind, wobei in der Körperhöhle eine erfindungsgemäße Vorrichtung angeordnet ist, bei der kelchartig verlaufende Mikrodrähte über ein Fluid auf die verletzte Gewebewand auftragen.
- Figur 6: eine Seitenansicht eines proximalen Endes eines medizinischen Endoskops, das eine endoskopische Einrichtung aufnimmt, und
- Figur 7: im Maßstab vergrößert, ein perspektivisch dargestelltes distales Ende des medizinischen Endoskops der Figur 6, wobei in dessen Arbeitskanal die erfindungsgemäße endoskopische Vorrichtung gemäß dem Ausführungsbeispiel nach der Figur 1 angeordnet ist

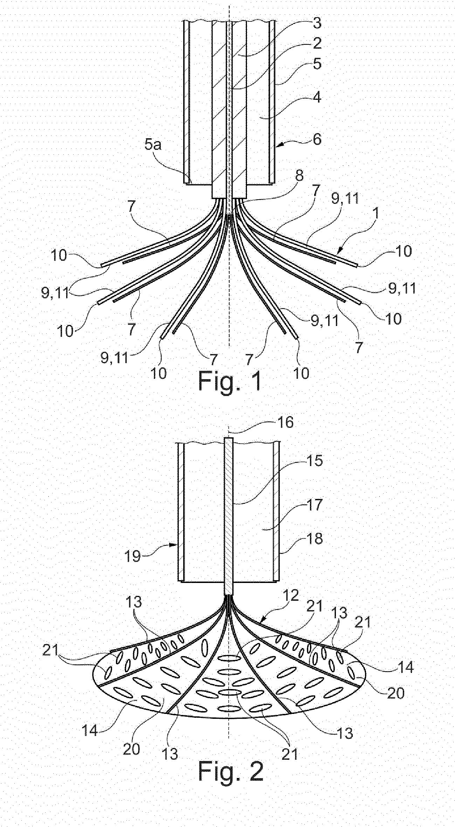

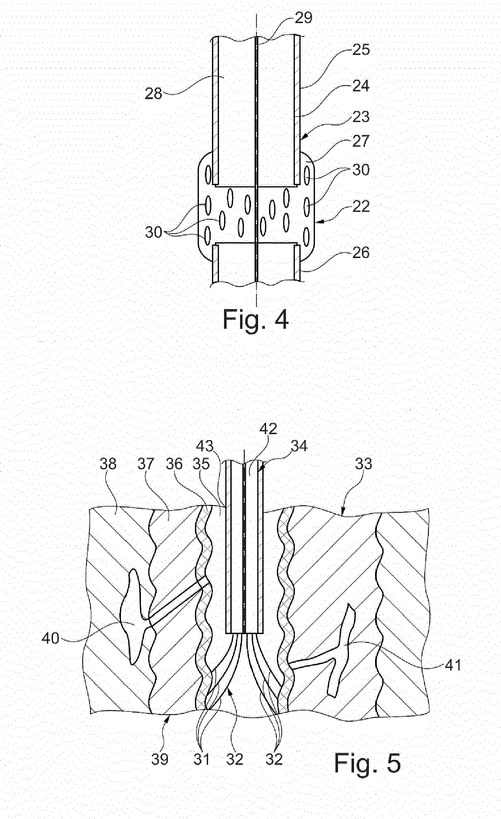

In der Figur 1 ist mit 1 eine Vorrichtung zur Applikation eines flüssigen therapeutischen Mittels an einer Gewebewand. Dabei umschließt die Gewebewand, wie nachfolgend noch im Zusammenhang mit der weiteren Figur 5 erläutert werden wird, eine Körperhöhle. Auch kann an die Körperhöhle ein menschliches Organ angrenzen.

Die Vorrichtung 1 ist mit einem als Betätigungsdraht oder -seil ausgebildeten Steuerelement 2 verbunden und von einem Hauptschlauch 3 umschlossen, wobei der Hauptschlauch 3 in einem Lumen 4 verläuft, das in einem Tubus 5 einer endoskopischen Einrichtung 6 ausgebildet ist. Die Vorrichtung 1 gemäß der Figur 1 besteht aus Mikrodrähten 7, die radial federnd ausgeführt und mit ihren proximalen Enden 8 an dem Steuerelement 2 fixiert sind. In der Darstellung nach der Figur 1 befinden sich die Mikrodrähte 7 in einem aus dem Lumen 4 distal ausgeschobenen Zustand, in dem sie sich kelchartig aufspreizen. Werden die Mikrodrähte 7 mittels des Steuerelements 2 in den Tubus 5 eingezogen, legen sie sich an einen Rand 5a eines distalen Endes des Tubus' 5 an und werden radial zusammengezogen.

An jedem der einzelnen Mikrodrähte 7 ist ein Zuführschlauch 9 fixiert, der jeweils in seinem Inneren einen distal mit zumindest einer Austrittsöffnung 10 endenden Zuführkanal 11 aufweist. Proximal sind die Zuführschläuche 9 mit dem Hauptschlauch 3 verbunden, wobei dieser mit einem nicht näher dargestellten Fluidkanal versehen ist, über das das flüssige Mittel in die einzelnen Zuführkanäle 11 gelangt, um an den distalen Enden der Zuführschläuche 9 aus diesen auszutreten.

Bei einem zweiten Ausgestaltungsbeispiel einer Vorrichtung 12 nach der Figur 2 besteht diese ebenfalls aus Mikrodrähten, die mit 13 bezeichnet sind und zwischen denen sich eine doppelwandige Folie 14 erstreckt. Einem zwischen den beiden Lagen der doppelwandigen Folie 14 gebildeten Zwischenraum wird das flüssige Mittel über einen Hauptschlauch 15 zugeführt, wobei der Hauptschlauch 15 zu diesem Zweck mit zumindest einem nicht näher dargestellten Fluidkanal versehen ist. Weiterhin verläuft innerhalb des Hauptschlauches 15 ein Steuerelement 16, das ebenfalls als Betätigungsdraht oder -seil ausgeführt sein kann.

Dieses Steuerelement 16 und ein den Hauptschlauch 15 über ein Lumen 17 aufnehmender Tubus 18 sind Bestandteile einer endoskopischen Einrichtung 19. Wie weiterhin der Figur 2 zu entnehmen ist, weist eine proximal gerichtete Folienlage 20 Austrittsöffnungen 21 für das Fluid auf, während eine in der Darstellung nicht sichtbare distal gerichtete Folienlage geschlossen ist. Die Vorrichtung 12, d.h., die Mikrodrähte 13 mitsamt der doppellagigen Folie 14 befinden sich in der Darstellung außerhalb des Lumens 17, so dass sie sich kelchartig aufgespreizt haben. Wenn die Vorrichtung 12 in das Lumen 17 eingezogen ist, werden die Mikrodrähte 13 gebündelt und die doppellagige Folie 14 gefaltet.

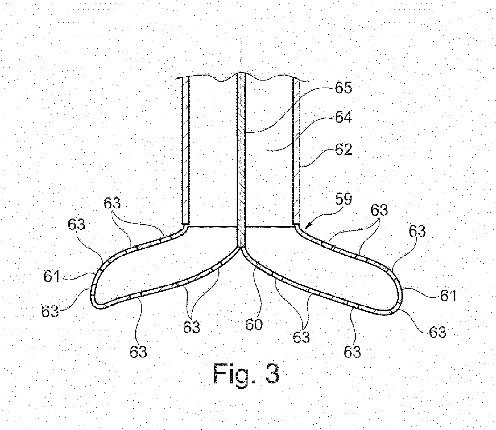

Eine alternative Ausführung einer Vorrichtung 59 zur Applizierung eines flüssigen therapeutischen Mittels zeigt die Figur 3. Dabei bildet eine Folie, die in Übereinstimmung mit der Folie 14 nach der Figur 2 ebenfalls die Form eines umgekehrten Trichters aufweist, einen Boden 60 und ist dabei einlagig ausgeführt. Vom umlaufenden Rand dieses Bodens 60 geht eine Wulst 61 aus, die mit dem distalen Ende eines Tubus' 62 verbunden ist. Sowohl in der Wulst 61 als auch im Boden 60 sind Austrittsöffnungen 63 für das Hämostasemittel ausgebildet. Über ein innerhalb des Tubus' 62 verlaufendes Lumen 64 wird das Hämostasemittel in einen vom Boden 60 und der Wulst 61 umschlossenen Raum 65 geleitet und tritt aus diesem über die Austrittsöffnungen 63 aus. Durch ein mittig an dem Boden 60 angreifendes mechanisches Steuerelement 65 lässt sich die Form der Wulst 61 und somit der Druck auf die Gewebewand verändern.

Ein drittes Ausgestaltungsbeispiel einer erfindungsgemäßen Vorrichtung 22 und einer an diese angepassten endoskopischen Einrichtung 23 gehen aus der Figur 4 hervor. Dabei besteht ein Tubus 24 aus einem langen proximalen Abschnitt 25 und einem kurzen distalen Endabschnitt 26, die über eine hülsenartig ausgebildete Folie 27 miteinander verbunden sind. Diese übergreift Enden des proximalen Abschnitts 25 und des distalen Endabschnitts 26 an deren Außenmantelflächen.

Ein innerhalb eines Lumens 28 verlaufendes Steuerelement 29 greift zumindest mittelbar an dem distalen Endabschnitt 26 an, so dass eine mittels des Steuerelements 29 auf diese übertragene Zugkraft zu einer Bewegung des Abschnitts 25 und des distalen Endabschnitts 26, in axialer Richtung aufeinander zu, führt. Dadurch verformt sich die Folie 27 wulstartig nach radial außen, so dass ein Druck auf die zu behandelnde Gewebewand ausgeübt werden kann, um das therapeutische Mittel oder Hämostasemittel, das über in der Folie 27 vorgesehene Austrittsöffnungen 30 aus dem Lumen 28 in die Oberfläche des Gewebes und/oder nach einer vorangegangenen Resektion als Wundverschluss in die entstandene Wunde in der Gewebewand gelangt. Da sich die hülsenartige Folie 27 sich radial wulstartig ausdehnt, wird das therapeutische Mittel bzw. Hämostasemittel in die zu behandelnde Gewebewand gedrückt.

In der Darstellung gemäß den Figuren 5 wird im Längsschnitt eine beispielhafte Verwendung einer Vorrichtung 31 gezeigt, bei der gegenüber dem Ausgestaltungsbeispiel nach der Figur 1 auf separate Zuführschläuche verzichtet und stattdessen nicht näher dargestellte Zuführkanäle innerhalb von Mikrodrähten 32 verlaufen. Diese sind an ihren distalen Enden offen. Teil des Längsschnittes ist auch ein Teil eines menschlichen Organs, beispielsweise eines Darms 33, in den die Vorrichtung 31 über eine endoskopische Einrichtung 34 eingeführt ist. Der Darm 33 bildet in seinem Inneren eine Körperhöhle 35, die von einer aus unterschiedlichen Gewebeschichten 36, 37 und 38 bestehenden Gewebewand 39 umschlossen ist. Bei der obersten Gewebeschicht 36 kann es sich folglich um eine Darmmukosa handeln.

Eine Resektion hat zu Wunden 40 und 41 in einigen dieser Gewebeschichten 36, 37, 38 geführt, wodurch Blutungen auftreten. Mittels der endoskopischen Einrichtung 34 wird die Vorrichtung 31 an der zu behandelnden Stelle im Darm 33 positioniert und die Mikrodrähte 32 in distaler Richtung aus einem Lumen 42 eines Tubus' 43 der endoskopischen Einrichtung 34 ausgeschoben, so dass sie sich kelchartig aufspreizen und mit ihren distalen Enden an der der Körperhöhle 35 zugewandten Oberfläche der Gewebewand 39 anliegen. Wenn das den Mikrodrähten 32 über den Tubus 43 zugeführte Hämostasemittel distal aus diesen austritt, wird die gesamte Vorrichtung 31 in proximaler Richtung bewegt, was zum Auftragen des Hämostasemittels auf die Gewebewand 39 führt. Dadurch wird erreicht, dass das Hämostasemittel in die Wunden 40 und 41 gelangt, diese versiegelt und somit die auftretenden Blutungen stillt.

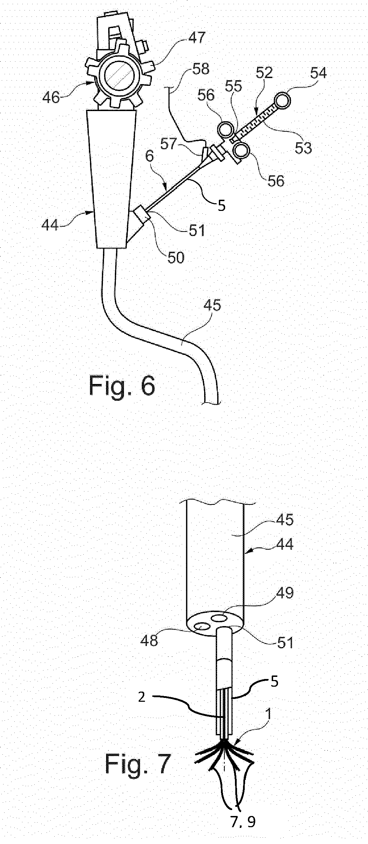

Die Figur 6 zeigt einen proximalen Teilabschnitt eines medizinischen Endoskops 44, mit dem sich beispielsweise bei einer Koloskopie des Darmes Untersuchungen in diesem durchführen lassen und mittels einer in dem Endoskop 44 geführten nicht dargestellten endoskopischen Einrichtung bei Bedarf eine Polypektomie zur Resektion von Polypen ausgeführt werden kann. Dabei ist in der Figur 6 von diesem Endoskop 44 nur ein proximaler Endabschnitt dargestellt, während die Figur 7, die im Maßstab gegenüber der Figur 6 vergrößert ist, ein distales Ende des medizinischen Endoskops 44 zeigt.

Gemäß der Figur 6 befindet sich an dem proximalen Ende eine mit einem Einführschlauch 45 verbundene Steuereinheit 46, die unter anderem aus einer Winkelsteuerung 47, mit der die Position des in der Figur 7 dargestellten distalen Endes des Einführschlauches 45, das eine Lichtquelle 48 und eine Kamera 49 aufnimmt, veränderbar ist. Weiterhin befindet sich an der Steuereinheit 46 eine Schleuse 50, über die die nach der Figur 1 ausgebildete erfindungsgemäße Vorrichtung 1 in einen im Inneren des Einführschlauches 45 verlaufenden Arbeitskanal 51 einführbar ist. Im vorliegenden Fall ist über die Schleuse 50 ein Tubus 5 nach der Figur 1 in den Arbeitskanal 51 des Endoskops 44 eingeführt und tritt, wie bereits erläutert, gemäß der Figur 7 am distalen Ende aus diesem aus. Der Tubus 5 mit dem Steuerelement 2 ist ebenso wie der Einführschlauch 45 verkürzt dargestellt.

An einem proximalen Ende des Tubus' 5 ist ein Betätigungselement 52 angeordnet, das einen am Tubus 5 über ein Führungselement 53 abgestützten Daumenring 54 aufweist. Dabei ist weiterhin auf dem Führungselement 53 eine Führungsbuchse 55 längsverschiebbar geführt, an der Fingerringe 56 fixiert sind. Die Führungsbuchse 55 greift an dem Steuerelement 2 an, so dass eine Verstellung des gemäß Figur 1 innerhalb des Tubus' 5 verlaufenden Steuerelements 2 bewirkt wird. Folglich lässt sich durch die Verschiebung der Führungsbuchse 55 über die Fingerringe 56 eine Verstellung der erfindungsgemäßen Vorrichtung mit ihren Mikrodrähten 7 und Zuführschläuchen 9 aus dem Lumen 4 heraus oder in dieses hinein bewirken.

Über die an der Führungsbuchse 55 angeordneten Fingerringe 56 kann der behandelnde Arzt mit einer Hand eine Verstellung der erfindungsgemäßen Vorrichtung 1 durchführen, wobei sein Daumen in den Daumenring 54 eingreift. Von der endoskopischen Einrichtung 6 geht eine Schleuse 57 aus, an die eine Leitung 58 zur Zuführung des Fluids angeschlossen ist.

Die gemäß der Figur 1 ausgeführte Vorrichtung 1 ist in der Figur 7 nur als Beispiel dargestellt. Es kann sich auch um eine der Vorrichtungen 12, 21, 31 oder 59 handeln, die jeweils in den Figuren 2, 3 oder 4 dargestellt ist.

### Bezugszeichenliste

- 1: Vorrichtung zur Applikation eines flüssigen therapeutischen Mittels
- 2: Steuerelement
- 3: Hauptschlauch
- 4: Lumen
- 5: Tubus
- 5a: distaler Rand von 5
- 6: endoskopische Einrichtung
- 7: Mikrodraht
- 8: Rand von 5
- 9: Zuführschlauch
- 10: Austrittsöffnung
- 11: Zuführkanal
- 12: Vorrichtung zur Applikation eines flüssigen therapeutischen Mittels
- 13: Mikrodrähte
- 14: doppelwandige Folie
- 15: Hauptschlauch
- 16: Steuerelement
- 17: Lumen
- 18: Tubus
- 19: endoskopische Einrichtung
- 20: außenliegende Folienlage von 14
- 21: Austrittsöffnungen
- 22: Vorrichtung zur Applikation eines flüssigen therapeutischen Mittels
- 23: endoskopische Einrichtung
- 24: Tubus
- 25: langer proximaler Abschnitt von 23
- 26: kurzer distaler Endabschnitt von 23
- 27: hülsenartige Folie
- 28: Lumen
- 29: Steuerelement
- 30: Austrittsöffnungen
- 31: Vorrichtung zur Applikation eines flüssigen therapeutischen Mittels
- 32: Mikrodrähte
- 33: Darm
- 34: endoskopische Einrichtung
- 35: Körperhöhle
- 36: Gewebeschicht
- 37: Gewebeschicht
- 38: Gewebeschicht
- 39: Gewebewand
- 40: Wunde
- 41: Wunde
- 42: Lumen
- 43: Tubus
- 44: medizinisches Endoskop
- 45: Einführschlauch
- 46: Steuereinheit
- 47: Winkelsteuerung
- 48: Lichtquelle
- 49: Kamera
- 50: Schleuse
- 51: Arbeitskanal
- 52: Betätigungselement
- 53: Führungselement
- 54: Daumenring
- 55: Führungsbuchse
- 56: Fingerringe
- 57: Schleuse von 6
- 58: Leitung zur Zuführung eines Fluids
- 59: Vorrichtung zur Applizierung eines flüssigen therapeutischen Mittels
- 60: Boden
- 61: Wulst von 60
- 62: Tubus
- 63: Austrittsöffnungen in 60 und 61
- 64: Lumen
- 65: mechanisches Steuerelement

## Patentansprüche

1. Vorrichtung (1, 12, 22, 31) zur Applikation eines therapeutischen, als Fluid ausgebildeten Mittels an einer Gewebewand (39), die eine Körperhöhle (35) umschließt, wobei die Vorrichtung (1, 12, 21, 30) an einem in die Körperhöhle (35) einführbaren Katheter angeordnet und nach dem Einführen in Bezug auf den Katheter zum Berühren der Gewebewand (35) radial aufspreizbar ist und wobei das über den Katheter zugeführte Fluid über Austrittsöffnungen (10, 21, 30) an die Gewebewand (39) gelangt, **dadurch gekennzeichnet, dass** der Katheter als Tubus (5,18, 24, 43) einer endoskopischen Einrichtung (6, 19, 23, 34) ausgebildet ist, wobei der Tubus (5,18, 24, 43) an seinem distalen Ende die Vorrichtung (1, 12, 22, 31) aufnimmt, dass der Tubus (5,18, 24, 43) ein Lumen (4, 17, 28, 42) aufweist, in welchem sich ein mechanisches Steuerelementelement (2, 16, 29) erstreckt, durch dessen proximal über ein Betätigungselement (52) bewirkten Längsbewegungen die Vorrichtung (1, 12, 22, 31) zwischen einem Zustand kleinster radialer Erstreckung und einem Zustand größter radialer Erstreckung verstellbar ist und dass die Vorrichtung (1, 12, 22, 31) zumindest in ihrem Abschnitt, der die Gewebewand (39) im aufgespreizten Zustand berührt, die Austrittsöffnungen (10, 21, 30) für das Fluid aufweist.

2. Vorrichtung nach Patentanspruch 1, **dadurch gekennzeichnet, dass** das Fluid ein Hämostasemittel zur Erzielung einer Hämostase an der Gewebewand (39) nach einer an dieser vorgenommenen Geweberesektion ist.

3. Vorrichtung nach Patentanspruch 1, **dadurch gekennzeichnet, dass** die in das Lumen (4, 17, 42) eingezogene Vorrichtung (1, 12, 31) in diesem Zustand radial federnd vorgespannt ist und nach ihrem Austritt aus dem Lumen (4, 17, 42), für den sie mittels des Steuerelements (2, 16) in distaler Richtung verschiebbar ist, in ihre radial aufgespreizte Stellung gelangt.

4. Vorrichtung nach Patentanspruch 3, **dadurch gekennzeichnet, dass** die Vorrichtung (1, 12, 31) durch Mikrodrähte (7, 13, 32) gebildet wird, die im aus dem Lumen (4, 17, 42) ausgeschobenen Zustand, insgesamt eine kelchartige Kontur bildend, zueinander verlaufen.

5. Vorrichtung nach Patentanspruch 4, **dadurch gekennzeichnet, dass** innerhalb der Mikrodrähte (32) Zuführkanäle verlaufen, deren Austrittsöffnungen zum Auftragen des Fluids auf die Gewebewand (39) dienen.

6. Vorrichtung nach Patentanspruch 4, **dadurch gekennzeichnet, dass** an den Mikrodrähten (7) Zuführschläuche (9) zum Auftragen des Fluids auf die Gewebewand (39) fixiert sind, deren Austrittsöffnungen (10) zum Auftragen des Fluids auf die Gewebewand (39) dienen.

7. Vorrichtung nach einem der Patentansprüche 5 oder 6, **dadurch gekennzeichnet, dass** die Zuführkanäle der Mikrodrähte (32) oder die Zuführschläuche (9) mit dem das Steuerelement (2, 16) umgebenden Tubus (5, 18,43) oder einem innerhalb des Lumens (4, 17, 42) verschiebbar geführten, das Steuerelement (2, 16) umschließenden Hauptschlauch (3) verbunden sind.

8. Vorrichtung nach Patentanspruch 4, **dadurch gekennzeichnet, dass** die Mikrodrähte (13) über eine doppelwandige Folienanordnung (14, 20) miteinander verbunden sind, wobei das Fluid zwischen den beiden Folienlagen geführt wird und über die in der äußeren Folienlage (20) vorgesehenen Austrittsöffnungen (21) auf die Gewebewand (39) übertragbar ist.

9. Vorrichtung nach Patentanspruch 1, **dadurch gekennzeichnet, dass** das Mittel der Vorrichtung (22) über das Lumen (28) oder eine in diesem angeordnete Versorgungsleitung zuführbar ist, wobei die Vorrichtung (22) durch eine hülsenartig verlaufende Folie (27) gebildet wird, die mit Austrittsöffnungen (30) für das Fluid versehen ist und Teilabschnitte (25 und 26) des Tubus' (24), die mittels des Steuerelements (29) in axialer Richtung relativ zueinander bewegbar sind, miteinander verbindet.

10. Vorrichtung nach Patentanspruch 1, **dadurch gekennzeichnet, dass** das Steuerelement (2, 16, 29) als Draht oder Seil ausgebildet ist.

11. Vorrichtung nach Patentanspruch 1, **dadurch gekennzeichnet, dass** die gemeinsam mit der endoskopischen Einrichtung (6, 19, 23, 34) ausgebildete Vorrichtung (1, 12, 22, 31) zur Applikation des als Fluid ausgebildeten Mittels für eine Anordnung in einem Arbeitskanal (51) eines medizinischen Endoskops (44) vorgesehen ist.
